Europäisches Patentamt

European Patent Office

Office européen des brevets

(11) Numéro de publication: **0 356 629**
**A1**

## (12) DEMANDE DE BREVET EUROPEEN

(21) Numéro de dépôt: 89110105.7

(51) Int. Cl.⁵: **A61B 8/08 , G01S 15/10**

(22) Date de dépôt: **03.06.89**

(30) Priorité: **13.06.88 FR 8807980**

(43) Date de publication de la demande:
**07.03.90 Bulletin 90/10**

(84) Etats contractants désignés:
**CH DE ES GB IT LI NL SE**

(71) Demandeur: **ASULAB S.A.**
**Faubourg du Lac 6**
**CH-2502 Bienne(CH)**

(72) Inventeur: **Farine, Pierre-André**
**Port-Roulant 12a**
**CH-2003 Neuchâtel(CH)**
Inventeur: **Bornoz, Claude**
**Rue des Acacias 10**
**CH-2114 Fleurier(CH)**
Inventeur: **Etienne, Jean-Daniel**
**Route de Vanel 31**
**CH-2206 Les Geneveys(CH)**

(74) Mandataire: **de Montmollin, Henri et al**
**ICB Ingénieurs Conseils en Brevets SA**
**Passage Max. Meuron 6**
**CH-2001 Neuchâtel(CH)**

(54) Suiveur d'écho pour appareil de mesure par ultrasons de la position d'une paroi mobile.

(57) Le suiveur d'écho recale une fenêtre temporelle
(AUT) de détection d'écho sur la position d'un écho
(ECHN) numérique obtenu à partir d'un écho (ECHA)
produit par une paroi mobile recevant un signal
ultrasonore d'impulsion à une fréquence de répétition $F_r$. Ce suiveur d'écho comprend un moyen
d'autorisation (20) qui définit la fenêtre temporelle,
un moyen de détection de position d'écho (22, 28)
pour détecter et mémoriser la position temporelle de
l'écho (ECHN), et un moyen d'asservissement (18,
28, 30) pour asservir la position de la fenêtre à la
position de l'écho détecté. Selon l'invention, le
moyen de détection est sensible à un front de sens
déterminé du signal d'écho.

Application dans le domaine médical à la mesure de la variation temporelle de la position d'une
interface entre deux tissus.

Fig. 4

Xerox Copy Centre

# SUIVEUR D'ECHO POUR APPAREIL DE MESURE PAR ULTRASONS DE LA POSITION D'UNE PAROI MOBILE

La présente invention concerne un suiveur d'écho pour un appareil de mesure par ultrasons de la position d'une paroi mobile.

L'invention trouve une application dans tous les domaines dans lesquels on souhaite suivre l'évolution temporelle de la position d'une paroi mobile, et notamment dans le domaine médical. Dans ce dernier, l'invention peut être utilisée pour suivre l'évolution temporelle de la position d'une interface entre deux tissus, et par exemple les profondeurs des parois antérieure et postérieure d'un vaisseau sanguin, pour déterminer l'évolution temporelle du diamètre de ce vaisseau sanguin.

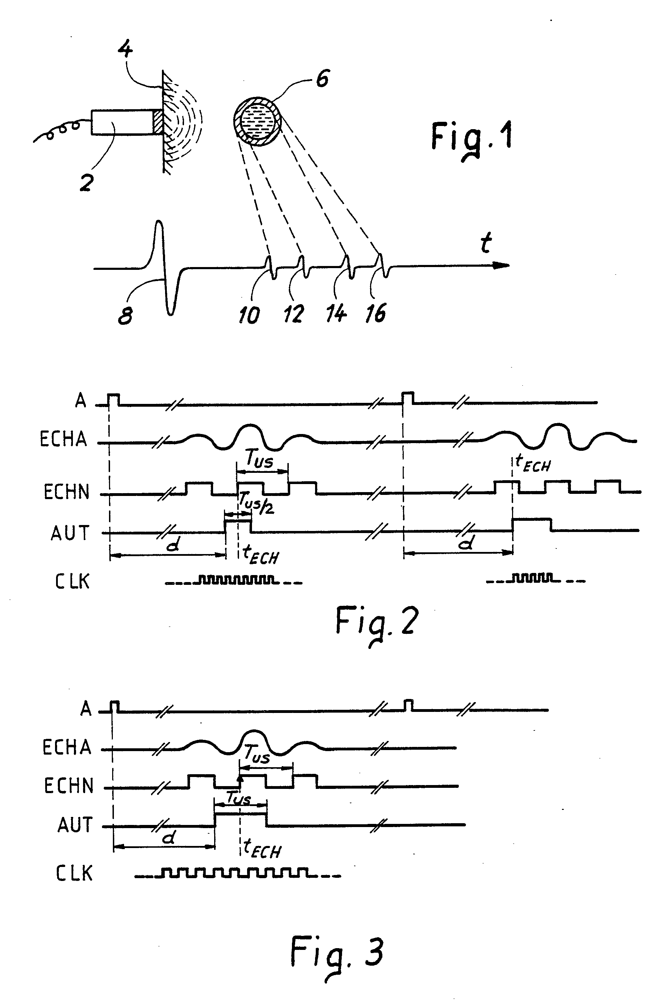

La figure 1 illustre schématiquement le principe connu de la mesure par ultrasons de la position d'une paroi mobile. Cette figure représente un transducteur d'onde ultrasonore 2 placé sur la peau 4 d'un sujet en face de l'artère radiale 6 représentée en coupe transversale. Le transducteur 2 est commandé par un circuit électronique pour émettre une impulsion d'onde ultrasonore 8 et pour recevoir les échos résultant de la réflexion de cette impulsion sur les interfaces artère-tissu ou artère-sang. Selon la fréquence du transducteur ultrasonore, on peut détecter quatre échos distincts 10, 12, 14, 16 ou seulement deux échos correspondant respectivement à une combinaison des échos 10 et 12, et à une combinaison des échos 14 et 16.

On détermine le mouvement d'une interface de la manière suivante. Le transducteur 2 émet une impulsion 8 avec une fréquence de répétition comprise généralement entre 100 Hz et 20 kHz. Pour suivre la position de l'écho, dont le retard sur l'impulsion 8 dépend de la position de l'interface, on utilise une fenêtre temporelle de largeur fixée qui définit un intervalle de temps dans lequel l'écho est attendu et qui est ouverte avec un retard ajustable après l'émission de l'impulsion 8, ce retard dépendant de la position de l'écho précédent. Le retard est ajusté, après chaque cycle, de manière que l'écho soit détecté par exemple au centre de la fenêtre si l'interface est immobile.

La connaissance de la position de chaque interface en fonction du temps permet, par différence, de déterminer l'évolution du diamètre du vaisseau sanguin 6 en fonction du temps.

La figure 1 illustre un schéma de principe. En pratique, le signal ultrasonore émis par le transducteur comporte au moins deux ou trois impulsions. Dans ce cas, il est clair que la même impulsion parmi la pluralité d'impulsions du signal d'écho doit être utilisée au cours des cycles successifs pour asservir la position de la fenêtre, car sinon la position de la paroi mobile n'est pas mesurée de manière fiable.

Les suiveurs d'écho récents sont de type numérique, c'est-à-dire que le signal d'écho reçu est un signal binaire. Un tel suiveur d'écho est décrit notamment dans l'article "A digital technique for tracking moving interfaces" de D.H. Groves et al, paru dans Ultra-sound Med. & Biol, vol. 8, no 2, pp 185-190, 1982.

Le chronogramme de la figure 2 illustre le fonctionnement de ce suiveur d'écho. Le signal A est appliqué au transducteur pour commander à intervalles réguliers l'émission d'un signal ultrasonore composé d'une suite de trois impulsions. L'écho analogique ECHA obtenu par réflexion sur la paroi mobile est converti en un écho numérique ECHN qui est appliqué au suiveur d'écho.

Celui-ci détecte la position de l'écho dans une fenêtre temporelle définie par le signal AUT; cette fenêtre a une largeur fixe et est ouverte avec un retard d sur l'impulsion de commande du signal A, la valeur de ce retard dépendant de la position de l'écho dans la fenêtre lors du cycle précédent.

La position de la fenêtre est asservie sur les positions d'une impulsion particulière du signal d'écho qui est de préférence l'impulsion la plus intense du signal d'écho ECHA, c'est-à-dire ici l'impulsion centrale.

Dans le suiveur d'écho décrit dans l'article cité, la position de l'écho est l'instant $t_{ECH}$ où le signal d'écho ECHN est au niveau logique haut dans la fenêtre définie par le signal AUT. Il s'ensuit que la fenêtre doit avoir une largeur maximale égale à $T_{us}/2$, où $F_{us} = 1/T_{us}$ est la fréquence du signal émis par le transducteur ultrasonore, comme on l'a représenté sur la moitié gauche de la figure. En effet, si la fenêtre a une largeur supérieure à $T_{us}/2$, elle peut contenir simultanément une partie au niveau logique haut de la première impulsion du signal d'écho et une partie au niveau logique haut de l'impulsion centrale du signal d'écho. Dans ce cas, représenté sur la moitié droite de la figure, le signal d'écho ECHN est au niveau haut dès le début de la fenêtre; l'instant de détection de l'écho $t_{ECH}$ est donc pris, de manière erronée, au début de la fenêtre sur le première impulsion du signal d'écho.

Il est important de noter également que le déplacement maximal de l'écho entre deux détections ne doit pas excéder $\pm 1/4 \, T_{us}$ pour que l'écho ne sorte pas de la fenêtre.

Dans les suiveurs d'écho numériques, la position de l'écho dans la fenêtre est généralement mesurée numériquement au moyen d'un signal d'horloge CLK. Ce signal a une fréquence $F_{CLK}$

suffisamment élevée pour que la fenêtre puisse avoir une largeur d'au moins trois périodes de ce signal de manière à pouvoir détecter si la paroi mobile s'est rapprochée (écho détecté plus tôt dans la fenêtre), si la paroi est immobile (écho détecté au milieu de la fenêtre), ou si la paroi mobile s'est éloignée (écho détecté plus tard dans la fenêtre).

Le fait de détecter l'écho sur un niveau logique haut associé à la condition d'un signal d'horloge ayant une période inférieure ou égale au tiers de la largeur de la fenêtre entraîne donc que l'on doit avoir : $F_{CLK} > 6 \cdot F_{us}$. Pour un transducteur ultrasonore à 10 MHz, il faut ainsi utiliser un signal d'horloge d'au moins 60 MHz. Ceci a pour désavantages d'induire une consommation électrique élevée et de nécessiter le recours à une technologie onéreuse.

La précision brute $\epsilon_b$ sur la position de l'écho dépend notamment de la fréquence d'horloge $F_{CLK}$. Cette précision peut être améliorée de manière connue en faisant un moyennage sur des ensembles de N valeurs de positions consécutives, la résolution effective $\epsilon_e$ atteinte étant alors égale à $\epsilon_b/N$. Le moyennage nécessite toutefois un signal d'horloge dont la période est au plus de 1/5e de largeur de la fenêtre, ce qui entraîne $F_{CLK} > 10 \cdot F_{us}$.

L'invention a pour but de réduire la fréquence minimale requise du signal d'horloge pour un transducteur de fréquence donnée. Ceci permet de réaliser un suiveur d'écho dans une technologie plus classique, donc moins onéreuse, et présentant une consommation électrique plus faible, sans que la résolution de l'appareil, liée à la fréquence ultrasonore, ne soit affectée.

Alternativement, ceci permet d'augmenter par rapport à l'art antérieur la fréquence du transducteur utilisable avec un signal d'horloge de fréquence déterminée. L'appareil a alors une meilleure résolution et peut ainsi distinguer deux parois plus proches.

L'invention consiste à détecter la position de l'écho sur un front de sens déterminé du signal d'écho. Il en résulte, comme on le verra dans la suite de la description, que la largeur de la fenêtre peut être doublée, ce qui diminue notablement la probabilité que l'écho sorte de la fenêtre, et que la fréquence minimale du signal d'horloge est divisée par deux, ce qui diminue la consommation électrique et permet d'utiliser une technologie moins coûteuse.

De manière précise, l'invention a pour objet un suiveur d'écho pour un appareil de mesure par ultrasons de la position d'une paroi mobile comprenant une entrée pour recevoir un signal numérisé d'écho, le signal d'écho étant produit par la réflexion sur ladite paroi mobile d'une impulsion ultrasonore d'interrogation émise à une fréquence de répétition $F_r$, ledit suiveur d'écho comprenant un moyen d'autorisation pour définir une fenêtre temporelle de longueur déterminée dans laquelle l'écho est attendu, un moyen de détection de l'écho pour détecter la position de l'écho dans ladite fenêtre, et un moyen d'asservissement pour asservir la position temporelle de ladite fenêtre à la position de l'écho détecté, ledit suiveur d'écho étant caractérisé en ce que ledit moyen de détection est sensible à un front de sens déterminé du signal d'écho numérisé.

Les caractéristiques et avantages de l'invention ressortiront mieux de la description qui va suivre, donnée à titre illustratif mais non limitatif, en référence aux dessins annexés, sur lesquels :

- la figure 1, déjà décrite, illustre le principe de la mesure par ultrasons de la position d'une paroi mobile,

- la figure 2, déjà décrite est un chronogramme illustrant le fonctionnement d'un appareil de mesure connu,

- la figure 3 est un chronogramme illustrant l'invention,

- la figure 4 représente un suiveur d'écho conforme à l'invention,

- la figure 5 est un chronogramme illustrant le fonctionnement du suiveur d'écho de la figure 4, et

- la figure 6 représente un appareil de mesure de la position d'une paroi mobile comportant deux suiveurs d'écho selon l'invention.

Le chronogramme de la figure 3 illustre l'invention. Il comprend un signal de commande A, un signal d'écho analogique ECHA et un signal d'écho numérisé ECHN qui sont respectivement identiques aux signaux analogues décrits en référence à la figure 2.

La position de la fenêtre est asservie sur la position d'une impulsion déterminée du signal d'écho qui, sur la figure, est l'impulsion centrale. Plus précisément, selon l'invention la position de l'écho est définie par la position $t_{ECH}$ d'un front de sens déterminé de cette impulsion. Sur la figure, on choisi à titre d'exemple le front montant de cette impulsion.

La distance entre deux fronts montants consécutifs étant égal à $T_{us}$, la largeur maximale de la fenêtre pour qu'elle ne contienne qu'un seul front montant, et éviter ainsi une détection erronée, est aussi égale à $T_{us}$.

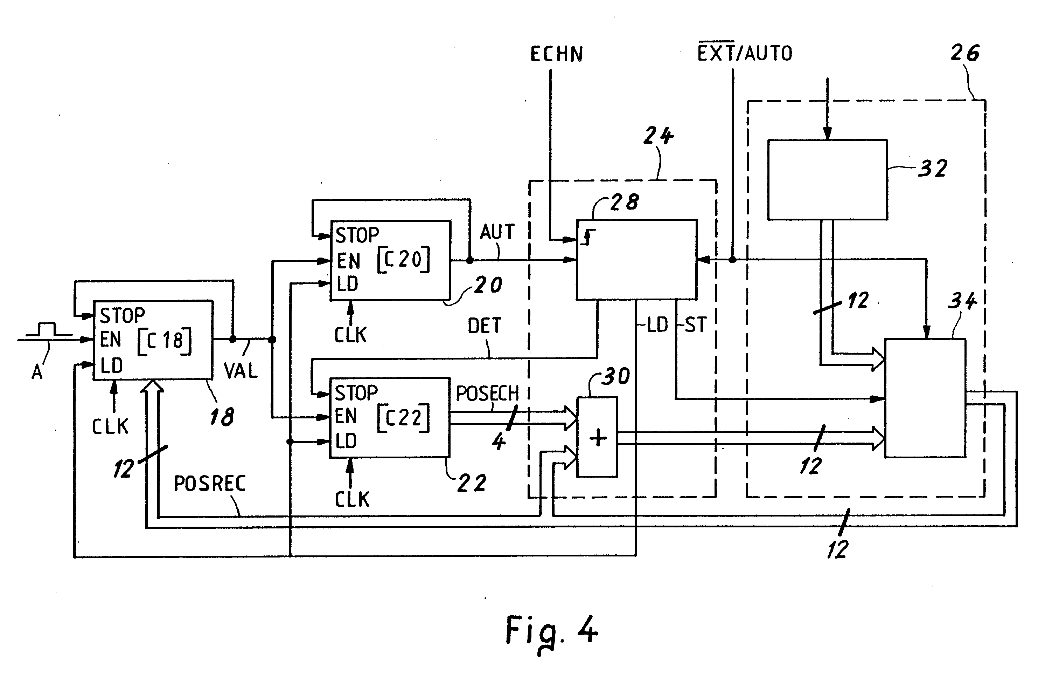

On a représenté sur la figure 4 un mode de réalisation d'un suiveur d'écho selon l'invention. Ce suiveur d'écho a pour fonction de détecter les échos correspondant à des impulsions ultrasonores émises avec une fréquence de répétition $F_r$ vers une paroi mobile. Ces impulsions ultrasonores sont commandées par le signal A (figure 3) qui est appliqué au suiveur d'écho et au transducteur ultrasonore. Pour chaque écho, la détection est autorisée dans une fenêtre temporelle de largeur déterminée dont la position est asservie sur l'instant de détection de l'écho précédant l'écho attendu. A cette fin, le suiveur d'écho comprend un compteur de profondeur 18 pour déterminer l'instant d'ouverture de la fenêtre temporelle, un moyen d'autorisation 20 pour définir la largeur de la fenêtre temporelle, un détecteur de position 22 pour mémoriser la position de l'écho reçu dans la fenêtre temporelle, et un circuit logique 24 pour détecter l'écho et commander le recalage de la fenêtre temporelle en fonction de la position de l'écho indiquée par le détecteur de position.

Le suiveur d'écho représenté sur la figure 4 comprend en outre un moyen de commande externe 26 pour modifier la position de la fenêtre temporelle indépendamment de la position de l'écho reçu. Ce moyen 26 est utilisé en phase d'initialisation ou, plus généralement, à chaque fois que les échos sortent accidentellement de la fenêtre.

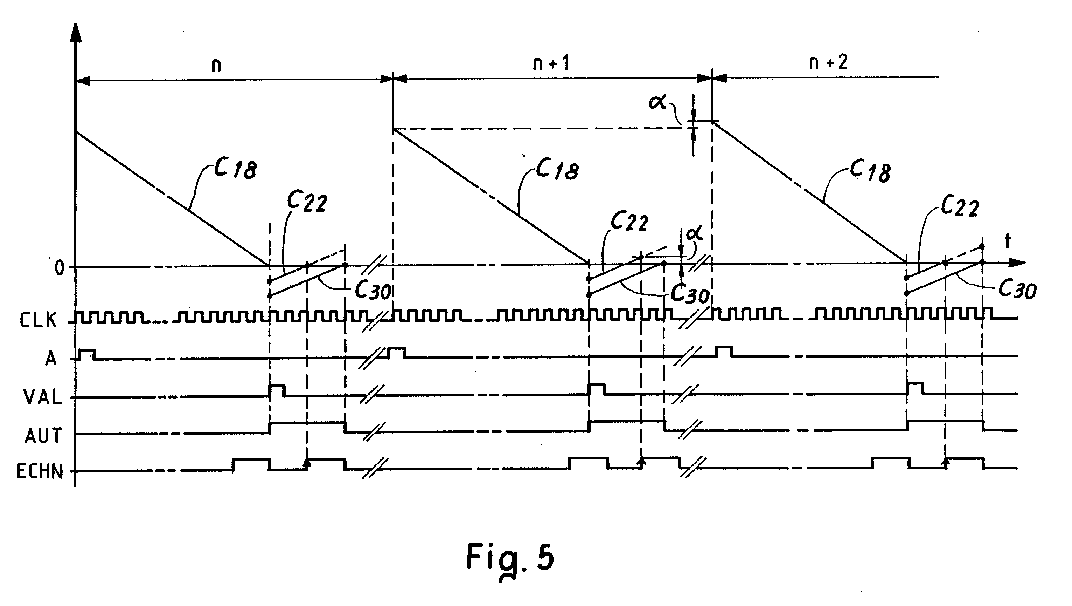

Le chronogramme de la figure 5 illustre le fonctionnement du suiveur d'écho. Celui-ci exécute des cycles successifs n, n + 1, n + 2... à la fréquence de répétition $F_r$ du signal de commande A. Avant le début de chaque cycle, le circuit logique 24 émet un signal LD pour charger des valeurs déterminées dans le compteur de profondeur 18, le moyen d'autorisation 20 et le détecteur de position 22.

La valeur POSREC chargée dans le compteur de profondeur 18 dépend de la position de l'écho dans la fenêtre. Cette valeur est stockée dans une mémoire 34 qui sera décrite ultérieurement. Les valeurs chargées dans le moyen d'autorisation 20 et le détecteur de position 22 sont des valeurs fixes contenues dans des mémoires non représentées sur la figure 4.

La valeur chargée dans le compteur de profondeur 18 est choisie de manière que ce compteur délivre le signal de validation VAL à l'instant où la fenêtre temporelle doit être ouverte. Ce signal de validation est émis lorsque le compteur contient une première valeur déterminée. On peut utiliser par exemple un compteur de profondeur muni d'une borne de sortie dont l'état logique est différent selon que la valeur contenue dans le compteur est égale à zéro ou est différente de zéro. Le signal de validation est alors obtenu directement sur cette borne de sortie. Le signal de validation peut également être constitué de manière simple par la valeur d'un bit de rang particulier du nombre binaire contenu dans le compteur. Il suffit en effet de charger initialement le compteur de profondeur avec une valeur choisie de manière à ce que le bit de rang sélectionné change d'état à l'instant où l'on désire ouvrir la fenêtre.

Le signal de validation délivré par le compteur de profondeur 18 sert principalement à activer le moyen d'autorisation 20 et le détecteur de position 22. Il peut être appliqué en outre sur une entrée STOP du compteur de profondeur 18 pour en arrêter le comptage.

Sur réception du signal de validation, le moyen d'autorisation 20 délivre un signal d'autorisation AUT de longueur déterminée qui définit la fenêtre temporelle pendant laquelle un écho est attendu. Ce moyen d'autorisation peut être réalisé par un compteur. Dans ce cas le signal d'autorisation peut être produit de la même manière que le signal de validation, c'est-à-dire qu'il peut être constitué par l'état logique d'un bit de rang déterminé du nombre binaire contenu dans le compteur, ou par l'état logique d'un bit ayant une valeur différente selon que le contenu du compteur est égal à zéro ou est différent de zéro.

Un autre mode de réalisation du moyen d'autorisation consiste à utiliser un registre à décalage. Dans ce mode de réalisation, le signal d'autorisation peut également être formé par l'état logique d'un bit de rang déterminé du registre à décalage ou par la valeur d'un bit dont l'état logique est différent selon que le contenu du registre à décalage est égal à zéro ou est différent de zéro.

Par exemple, pour produire un signal d'autorisation à l'état haut pendant 5 périodes du signal d'horloge CLK, on peut choisir pour signal d'autorisation la valeur du bit de poids le plus faible d'un registre à décalage de 8 bits chargé avec la valeur "00011111". En effet, ce bit vaut "1" pendant les 5 premiers décalages à droite commandés par le signal CLK puis passe à "0" à partir du 6ème décalage à droite.

Le détecteur de position 22 est activé par le signal de validation émis par le compteur de profondeur 18. Son comptage est arrêté par un signal de détection délivré par l'unité 28 du circuit logique 24 sur une entrée STOP du détecteur de position 22 à l'instant où le signal d'écho apparaît dans la fenêtre temporelle.

Conformément à l'invention, la détection de l'écho consiste à rechercher un front de sens déterminé d'une impulsion particulière du signal d'écho. Dans l'exemple représenté, on détecte le front montant de la deuxième impulsion (figure 5).

Dans le mode de réalisation de la figure 4, c'est l'unité logique 28 qui détecte le signal d'écho et qui vérifie qu'il apparaît dans la fenêtre temporelle. La détection peut être réalisée par exemple au moyen d'une bascule bistable de type 74AC74 recevant sur son entrée d'horloge CLK le signal d'écho ECHN et la vérification peut être réalisée simplement par une porte logique ET.

Il est clair que l'on pourrait également appliquer le signal d'écho directement sur l'entrée d'arrêt STOP du détecteur de position, en choisissant dans ce cas un détecteur de position dont l'entrée d'arrêt STOP est sensible à un front montant d'impulsion, et vérifier ensuite avec l'unité logique si la valeur contenue dans le détecteur de position correspond à un écho reçu dans ou hors de la fenêtre temporelle.

La valeur POSECH contenue dans le détecteur de position 22 représente le déplacement à appliquer à la fenêtre pour la recentrer sur l'écho reçu. Ce déplacement est réalisé par une mise à jour de la valeur chargée par le circuit logique 24 dans le compteur de profondeur 18. Cette mise à jour est particulièrement simple si la valeur initiale chargée dans le détecteur de position 22 au début du cycle est choisie de manière que, dans le cas d'une paroi immobile, le détecteur de position contient la valeur zéro lorsque l'écho est détecté. La valeur chargée dans le compteur de profondeur est alors modifiée simplement en lui ajoutant la valeur contenue dans le détecteur de position 22. Ceci est réalisé par un additionneur 30. Cette valeur de chargement du compteur de profondeur est mémorisée dans une mémoire 34 dont l'accès est autorisé par un signal ST émis par l'unité logique 28.

Le chronogramme de la figure 5 illustre les principaux signaux apparaissant sur la figure 4, ainsi que les valeurs contenues dans les compteurs. Les références C18, C20 et C22 correspondent respectivement aux contenus du compteur de profondeur 18, d'un compteur formant le moyen d'autorisation 20, et du détecteur de position 22.

Chaque cycle de mesure commence avec une impulsion de commande du signal A. Cette impulsion fait débuter le comptage du compteur 18 jusqu'à une première valeur déterminée (égale à zéro sur la figure 5) qui provoque l'émission du signal de validation VAL.

Ce signal active le moyen d'autorisation 20 et le détecteur de position 22. Le signal d'autorisation AUT, qui définit la fenêtre temporelle, s'affirme pendant une durée prédéterminée qui, sur la figure 5, est égale à 5 périodes du signal CLK. Cette durée est définie par la durée d'un comptage jusqu'à une valeur déterminée qui est égale à zéro sur la figure.

A partir du début de la fenêtre temporelle, le contenu C22 du détecteur de position 22 évolue jusqu'à l'apparition de l'écho. La valeur à cet instant reflète la position de l'écho dans la fenêtre, et donc la valeur de recalage de position à appliquer à la fenêtre.

Sur la figure, la valeur chargée dans le détecteur de position 22 est choisie pour que, dans le cas d'une paroi immobile, l'écho soit détecté lorsque le détecteur de position contient la valeur zéro. Ceci permet de recaler très facilement la fenêtre comme le montrent les cycles successifs n, n+1, n+2.

Au cours du cycle n, l'écho apparait au centre de la fenêtre (C22 est égal à zéro). La fenêtre est donc en position correcte et le compteur de profondeur 18 est chargé pour le cycle n+1 avec la même valeur que pour le cycle n. La fenêtre temporelle apparait donc au même instant, par rapport au début du cycle défini par l'impulsion de commande A, au cours des cycles n et n+1.

Dans le cycle n+1, l'écho apparait plus tard dans la fenêtre, montrant que la paroi mobile s'est éloignée de l'appareil de mesure. Ce retard se traduit par une valeur α supérieure à zéro dans le détecteur de position. Pour recaler la fenêtre, cette valeur α est ajoutée à la valeur chargée dans le compteur de profondeur 18 pour le cycle n+2.

Si au contraire la paroi mobile se rapproche de l'appareil de mesure, l'écho apparait avant le milieu de la fenêtre et le détecteur de position contient alors une valeur négative. Il suffit également d'ajouter cette valeur négative pour recentrer la fenêtre. Bien entendu, on peut remplacer le compteur 22 par un décompteur et, dans ce cas, l'additionneur est remplacé par un soustracteur.

Sur la figure 5, on note que les signaux A et CLK sont indépendants. Ceci n'est pas nécessaire pour le fonctionnement du suiveur d'écho mais permet, en aval, de faire un moyennage sur les valeurs de positions de la paroi mobile. Il en résulte une augmentation de la précision comme on le verra dans la suite de la description.

En mode de fonctionnement normal, la position de la fenêtre est recalée automatiquement en fonction de la position des échos. Cependant, il est possible que, de manière accidentelle, la fenêtre ne soit plus calée sur le signal d'écho, par exemple si le signal d'écho disparait pendant un certain temps et que la paroi mobile se déplace. Par ailleurs, à la mise en marche de l'appareil, l'écho n'apparait pas nécessairement dans la fenêtre. Pour ces raisons, le suiveur d'écho représenté sur la figure 4 est pourvu d'un moyen de commande externe de la position de la fenêtre.

Ce moyen de commande externe 26 comprend un compteur 32 et une mémoire 34 à deux entrées multiplexées. La valeur contenue dans le compteur 32 peut être modifiée par un signal externe de commande de comptage ou de décomptage. La valeur du compteur 32 est appliquée sur une entrée de la mémoire 34. L'autre entrée de cette mémoire reçoit la valeur délivrée par l'additionneur 30.

La sélection entre le mode manuel et le mode automatique de calage de la fenêtre est défini par un signal externe EXT/AUTO qui sélectionne l'une des entrées de la mémoire 34. Ce signal est également appliqué à l'unité logique 28 qui délivre le signal ST d'accès à la mémoire 34.

A titre d'exemple, un suiveur d'écho conforme à la figure 4 et fonctionnant avec une horloge CLK à 60 MHz peut comprendre des compteurs à 12 bits de type 74ACT191 pour le compteur de profondeur 18, le moyen d'autorisation 20, le détecteur de position 22 et le compteur 32, un additionneur de type 74HCT283 pour l'additionneur 30, et un ensemble comprenant une mémoire de type 74HCT174 et un multiplexeur de type 74HCT604 pour la mémoire 34. En ce qui concerne l'unité logique 28, on peut utiliser un ensemble de portes logiques et de bascules bistables bien connues des spécialistes, et notamment une bascule bistable 74AC74 pour détecter l'apparition du front attendu dans le signal d'écho.

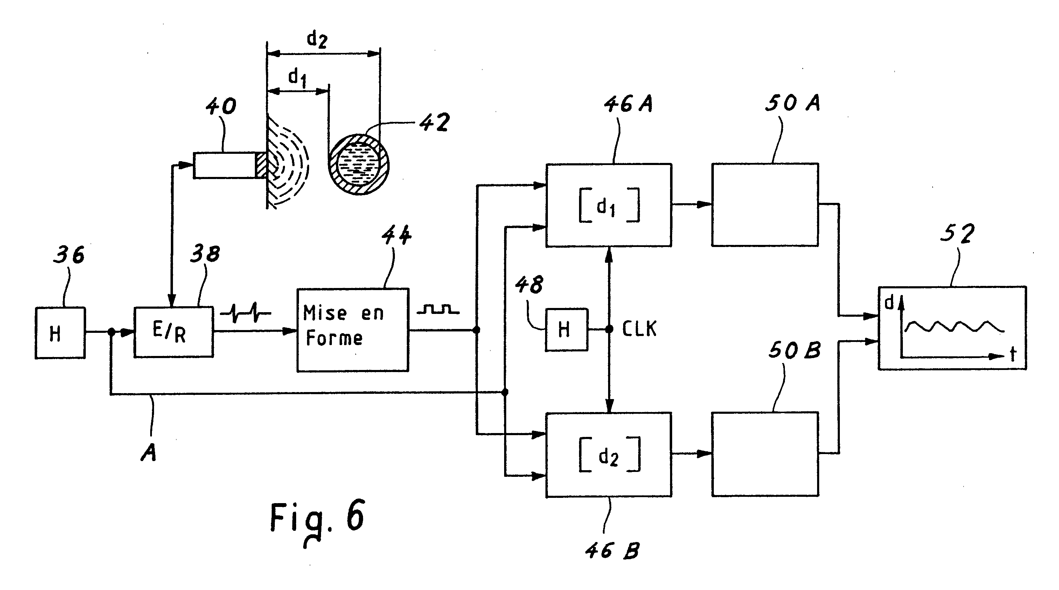

Grâce à l'invention, qui permet de diviser par deux la fréquence requise du signal d'horloge pour une fréquence ultrasonore donnée, le suiveur d'écho décrit présente une consommation électrique réduite, et peut être réalisé dans une technologie peu coûteuse (ACT/HCT au lieu de ECL), tout en ayant des performances élevées. Ce suiveur d'écho est un élément d'un appareil de mesure par ultrasons de la position d'une paroi mobile. On a représenté schématiquement sur la figure 6 un mode de réalisation d'un tel appareil, qui est appliqué dans le domaine médical à la mesure du diamètre d'une artère.

Cet appareil comprend une horloge 36 délivrant un signal d'impulsion à une fréquence de répétition de 5 kHz. Ce signal est reçu par un circuit d'émission-réception 38 qui commande un transducteur ultrasonore 40. Celui-ci émet une impulsion de fréquence 10 MHz vers l'artère 42 et recueille les échos réfléchis par les parois antérieure et postérieure de cette artère. Ces échos sont délivrés par le circuit 38 à un moyen de mise en forme 44 comprenant de manière connue des moyens de filtrage et un convertisseur analogique - numérique.

Le signal numérique délivré par le moyen de mise en forme 44 est appliqué sur les entrées de deux suiveurs d'échos 46A, 46B qui sont chacun conformes au suiveur d'écho représenté sur la figure 4. Les suiveurs d'échos reçoivent un même signal d'horloge CLK de fréquence 60 MHz délivré par une horloge 48. En pratique, chaque suiveur d'écho peut également être réalisé sur une carte indépendante comprenant une horloge CLK. Dans ce cas, chaque suiveur d'écho possède sa propre horloge. Ces horloges n'ont pas besoin d'être synchronisées.

Les fenêtres temporelles des suiveurs d'échos 46A et 46B sont calés respectivement sur l'écho produit par la paroi antérieure et l'écho produit par la paroi postérieure de l'artère 42. Chaque suiveur d'écho délivre donc, à la fréquence de répétition $F_r$, la position de la paroi de l'artère sur laquelle il est calé, c'est-à-dire la distance entre cette paroi et le transducteur 40.

La suite des positions délivrée par chaque suiveur d'écho est reçue dans un circuit de moyennage 50A, 50B. Chaque circuit permet, de manière connue, d'augmenter la précision de la mesure en calculant la valeur moyenne d'un ensemble de valeurs de positions consécutives ou en faisant une moyenne glissante sur un ensemble de valeurs de position consécutives.

On sait en effet que la résolution brute $\epsilon_b$ sur la position d'une paroi mobile est égale à $c/(2\ F_{CLK})$, où c est la vitesse de propagation du son dans le milieu situé entre le transducteur et la paroi, et $F_{CLK}$ la fréquence du signal CLK, alors que la résolution effective $\epsilon_e$ après moyennage sur des ensembles de N positions consécutives est égale à $\epsilon_b/N$. Cette opération de moyennage n'est possible que si les horloges 36 et 48 sont indépendantes.

Les valeurs délivrées par les circuits de moyennage 50A, 50B sont reçues dans un circuit 52 d'exploitation des résultats. Ce circuit peut comprendre une unité de mémorisation et une unité d'affichage. Il peut comprendre également une unité de calcul pour calculer le diamètre de l'artère 42 au cours du temps par différence des positions des parois antérieure et postérieure.

De manière avantageuse, le circuit 52 peut comprendre un micro-ordinateur. Dans ce cas, le moyennage peut être réalisé aisément par logiciel.

**Revendications**

1.- Suiveur d'écho pour appareil de mesure par ultrasons de la position d'une paroi mobile comprenant une entrée pour recevoir un signal numérisé d'écho (ECHN), le signal d'écho (ECHA) étant produit par la réflexion sur ladite paroi mobile d'une impulsion ultrasonore d'interrogation émise à une fréquence de répétition $F_r$, ledit suiveur d'écho comprenant un moyen d'autorisation (20) pour définir une fenêtre temporelle (AUT) de longueur déterminée, dans laquelle l'écho est attendu, un moyen de détection de position de l'écho (22, 28) pour détecter l'écho et déterminer sa position temporelle dans ladite fenêtre, et un moyen d'asservissement (18, 28, 30) pour asservir la position temporelle (POSREC) de ladite fenêtre à la position (POSECH) de l'écho détecté, ledit suiveur d'écho étant caractérisé en ce que ledit moyen de détection (28) est sensible à un front de sens déterminé du signal d'écho numérisé (ECHN).

2. Suiveur d'écho selon la revendication 1 caractérisé en ce que le moyen de détection comprend une bascule bistable, ladite bascule bistable comprenant une entrée d'horloge sur laquelle est appliquée ledit signal d'écho numérisé.

3. Suiveur d'écho selon l'une quelconque des revendications 1 et 2 comprenant un circuit d'horloge (48) délivrant un signal d'horloge CLK au moyen d'autorisation (20), au moyen de détection (22, 28) et au moyen d'asservissement (18, 28, 30) caractérisé en ce que le moyen d'autorisation (20) définit une fenêtre temporelle ayant une largeur au moins égale à trois périodes du signal d'horloge.

4. Suiveur d'écho selon l'une quelconque des revendications 1 et 2 comprenant un circuit d'horloge (48) délivrant un signal d'horloge CLK au moyen d'autorisation (20), au moyen de détection (22, 28) et au moyen d'asservissement (18, 28, 30) ledit signal d'horloge étant indépendant du signal d'interrogation, caractérisé en ce que le moyen d'autorisation (20) définit une fenêtre temporelle ayant une largeur égale à cinq périodes du signal d'horloge.

5. Suiveur d'écho selon l'une quelconque des revendications 1 à 4 caractérisé en ce que le moyen d'asservissement (18, 28, 30) commande le moyen d'autorisation pour que, dans le cas où la paroi est immobile, l'écho soit détecté au milieu de la fenêtre temporelle.

Fig.1

Fig.2

Fig. 3

Fig. 4

EP 0 356 629 A1

Fig. 5

EP 0 356 629 A1

Fig. 6

EP 0 356 629 A1

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | EP-A-0 185 133 (INSTITUT F. FOERSTER PRUEFGERAETEBAU GMBH & CO KG) <br> * figure 3; revendication 1 * <br> --- | 1 | A 61 B 8/08 <br> G 01 S 15/10 |
| Y | US-A-4 370 985 (H. TAKEICHI et al.) <br> * figures 4,5; colonne 3, ligne 31 - colonne 5, ligne 15 * <br> --- | 1 | |
| A | DE-A-2 853 170 (K. DEUTSCH PRUEF- UND MESSGERAETEBAU) <br> * figure 1; page 13, ligne 17 - page 4, ligne 14 * <br> --- | 1 | |
| D,A | ULTRASOUND IN MEDICINE AND BIOLOGY vol. 8, no. 2, 1982, pages 185-190, Oxford, GB; D. H. GROVES et al.: "A Digital Technique for Tracking Moving Interfaces" * page 186; figure 2 * <br> --- | | |
| A | US-A-4 721 113 (G. J. STEWART et al.) <br> * abrégé * <br> ----- | | |

**DOMAINES TECHNIQUES RECHERCHES (Int. Cl.4)**

G 01 S
A 61 B
G 01 B

Le présent rapport a été établi pour toutes les revendications

| Lieu de la recherche | Date d'achèvement de la recherche | Examinateur |
|---|---|---|
| BERLIN | 08-09-1989 | BREUSING J |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)